# EUROPEAN PATENT APPLICATION

(11) **EP 1 854 541 A1**
(43) Date of publication of application: **14.11.2007**
(21) Application number: 06405199.8
(22) Date of filing: 12.05.2006
(51) Int. Cl.: B01L 3/00

(54) **Multi-well plate**

(71) Applicant: F. Hoffmann-la Roche AG, 4070 Basel (CH)
(72) Inventor: Dr. Hochstrasser, Remo Anton, 4104 Oberwil (CH); Voegelin, Dieter, 4450 Sissach (CH)
(74) Representative: Heinen, Detlef

(57) **Abstract**

A multi-well plate (1) for mixing a fluid, comprises a plurality of wells (2). Each of the wells (2) has an opening arranged at a top side of the multi-well plate (1). At least one of the wells (2) has a cross-section parallel to the top side having an elongated shape. By means of such a multi-well plate (1) a faster and therefore more efficient mixing is possible using standard mixing devices such as shakers.

## Description

### Technical field

The present invention relates to a multi-well plate for mixing a fluid, for example a suspension and in particular a cell suspension, in general and more particularly to a system for mixing a fluid.

### Background art

Particularly in chemical, microbiological and pharmaceutical industry, as well as in chemical, microbiological and pharmaceutical research, standardized microplates are commonly used. In particular, microplate standards developed by the Society for Biomolecular Screening (SBS) and approved by the American National Standards Institute (ANSI) define microplates of 127.76 mm length, 85.48 mm width and 14.35 mm height comprising 96, 384 or 1536 wells [see Society for Biomolecular Screeneing. ANSI/SBS 1-2004: Microplates - Footprint Dimensions, ANSI/SBS 2-2004: Microplates - Height Dimensions, ANSI/SBS 3-2004: Microplates - Bottom Outside Flange Dimensions and ANSI/SBS 4-2004: Microplates - Well Positions. http://www.sbsonline.org: Society for Biomolecular Screeneing, 2004. ]. The wells of said standardized microplates usually have a circular or square shaped cross-section and a flat, rounded or pyramidal, symmetrical bottom.

In use the, wells can be filled with fluids, such as chemical or microbiological assays comprising several components. In order to establish homogenous conditions, frequently said fluids are mixed in the wells. For mixing, stirrers and shakers are widespread, wherein shakers, as for example VARIOMAG® TELESHAKE, are usually preferred especially in biological assays because stirrers may adversely affect the fluid or the analytical readout.

With regard to standardized microplates having 96 wells, common shakers are usually sufficient for fast and efficient mixing of fluids. Furthermore, using standardized microplates with 1536 wells, diffusion processes normally allow adequate mixing of fluids. In contrast to this, the mixing effect of shakers as well as of diffusion processes is frequently dissatisfying in standardized microplates having 384 wells.

To improve mixing by shakers in standardized microplates, it is known to insert blades into the wells while shaking. However, such blades are usually not preferred since they may adversely affect the fluid or the analytical readout and since they complicate the procedure as the blades have to be cleaned before each use. Otherwise, if blades are used for several fluids, or the blades are used in several microplates there is a risk of cross-contamination.

Therefore there is a need for a multi-well plate allowing efficient mixing of a fluid arranged in a well which overcomes the above described disadvantages.

### Disclosure of the invention

According to the invention this need is settled by a multi-well plate for mixing a fluid as it is defined by the features of independent claim 1, and by a system for mixing a fluid as it is defined by independent claim 6. Preferred embodiments are subject of the dependent claims.

In particular, the invention deals with a multi-well plate for mixing a fluid, comprising a plurality of wells. Each of the wells has an opening arranged at a top side of the multi-well plate and at least one of the wells has a cross-section parallel to the top side having an elongated shape. Elongated shape in the sense of the invention comprises all geometrical forms being suitable for the use as described below.

An advantage of such a well is that mixing of a fluid inside the well by means of a shaker can be improved. Suitable shakers for mixing fluids are widespread available in various embodiments, as for example VARIOMAG® TELESHAKE, particularly for the use of standardized microplates, e.g. comprising 96, 384 or 1536 wells. Compared to a round or squared cross-section of a well, an elongated cross-section allows to provide a more turbulent mixing movement using one of said shakers. Thus, particularly if a mid-sized well is used, e.g. a well of a standardized microplate comprising 384 wells, a faster and therefore more efficient mixing is possible.

Another advantage of said elongated cross-section is that a compact arrangement is possible. Particularly, if standardized microplates comprising 96, 384 or 1536 wells are used, such compact arrangement can be essential.

Preferably, the cross-section of said at least one of the wells of the multi-well plate has an oval shape or the shape of a rounded rectangle. Said shapes provide above described advantages for the well while they are comparably easy to fabricate.

The bottom of said at least one of the wells can be slanted. Such a slanted bottom allows a further improvement of mixing a fluid inside the well by means of a shaker. Since the slanted bottom causes the deepest point of the well to be arranged at one longitudinal end region of the elongate cross-section a fluid inside the well is comparably easy accessible to extraction means.

Preferably, the bottom of said at least one of the wells is rounded. A rounded bottom allows to lower the dead volume of a fluid in the well and it allows to further improve the complete extraction of the fluid out of the well. Additionally, it allows to still further improve the mixing of a fluid inside the well by means of a shaker

In a preferred embodiment the multi-well plate comprises 384 wells arranged in 16 rows and 24 columns. Particularly for such an arrangement of the wells within a standardized microplate having a length of 127.76 mm, a width of 85.48 mm and a height of 14.35 mm, the multi-well plate according to the invention provides an essentially improved mixing efficiency.

In another preferred embodiment the multi-well plate comprises 96 wells arranged in 8 rows and 12 columns. The multi-well plate according to the invention provides also an essentially improved mixing efficiency for such an arrangement of the wells within a standardized microplate having a length of 127.76 mm, a width of 85.48 mm and a height of 14.35 mm.

Preferably, the multi-well plate according to the invention is made by injection molding of an appropriate material such as polystyrene, polypropylene, acrylonitrile butadiene styrene copolymer (ABS) or cyclo olefin copolymer (COC).

A second aspect of the invention deals with a system for mixing a fluid, comprising the multi-well plate described above and a mixing device arranged for shaking the multi-well plate. In particular, the mixing device can be a standard shaker, as for example VARIOMAG® TELESHAKE, commonly used for mixing fluids in standardized microplates.

### Brief description of the drawings

The multi-well plate according to the invention is described in more detail hereinbelow by way of an exemplary embodiment and with reference to the attached drawings, in which:

Fig. 1 shows a perspective view onto a multi-well plate according to the invention;

Fig. 2 shows a top view onto the multi-well plate of Fig. 1;

Fig. 3 shows a cross-sectional view along the line B-B of the multi-well filtration device of Fig. 2; and

Fig. 4 shows a cross-sectional view along the line A-A of the multi-well filtration device of Fig. 2.

### Mode(s) for carrying out the invention

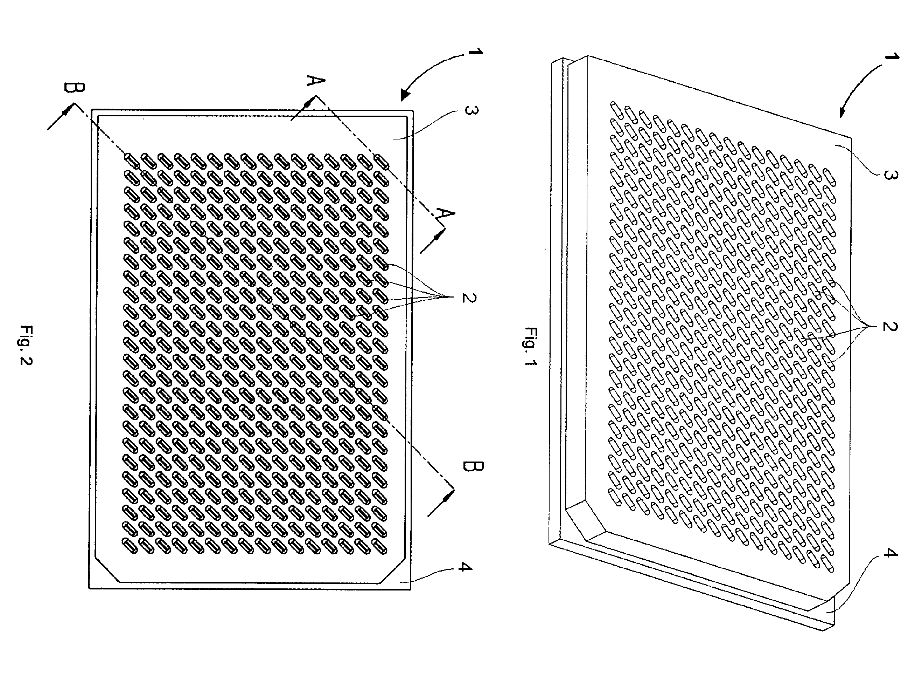

Fig. 1 and Fig. 2 show views of a multi-well plate 1 according to the invention. The multi-well plate 1 comprises a well layer 3 arranged onto a socket 4. It further comprises 16 rows and 24 columns of wells 2 each of them having an elongated cross section. The longitudinal directions of the elongated cross sections run in angle of 45 degrees to the longitudinal direction of the multi-well plate 1. The 384 wells 2, the well layer 3 and the socket 4 are arranged in a manner, such that the multi-well plate 1 is compliant to the ANSI approved microplate standards (see above). The angle of 45 degrees allows an optimal utilization of space.

For the further description of the exemplary embodiment using the figures, the following applies: If a figure contains reference signs which are not explained in the direct corresponding part of the description, they are explained in a preceding part of the description.

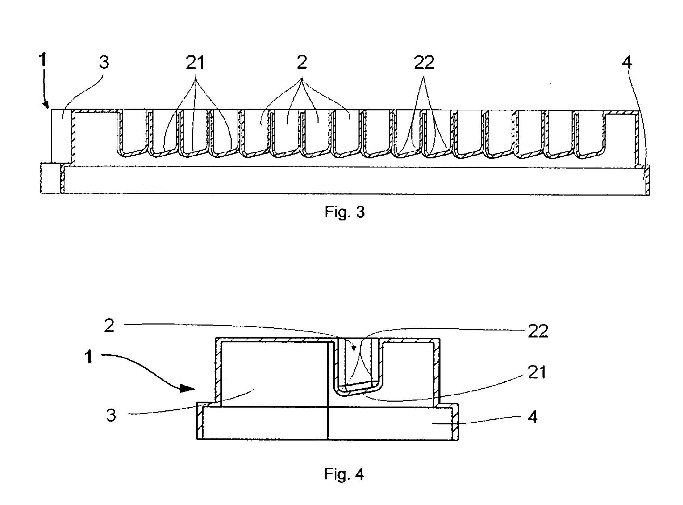

Fig. 3 shows a cross-sectional view in longitudinal direction of the plurality of wells 2 along the line B-B of Fig. 2. The wells 2 are fully arranged inside the well layer 3 of the multi-well plate 1. The bottom of each of the wells 2 has a slanted part 21 ending in rounded parts 22.

As best seen in Fig. 4 showing a diagonal cross-sectional view in longitudinal direction of one single well 2 along the line C-C of Fig. 2, the bottom of each of the wells has rounded parts 22 being arranged adjacent to the slanted part 21 of the bottom in all directions.

In use, for extracting a fluid out of one of the wells 2, extracting means, such as for example the tip of an extracting needle, can be arranged at the deepest point of the well 2. Thus, the dead volume in the well 2 can be efficiently minimized.

### Example

The improved mixing efficiency of the multi-well plate according to the invention is described hereinbelow with the help of mixing experiments in a challenging test system. The following tables show the results of said mixing experiments, wherein mixing times have been measured on one hand using multi-well plates according to the invention and on the other hand using regular 384 well standardized microplates with wells having circular cross-sections. First, at the bottom of the wells a fixed amount of a coloured solute, i.e. Bromophenol in 10% glycerol / 90% water, has been arranged followed by a varying amount of solvent (water). Second, the multi-well plates or the microplates, respectively, have been shaken for a certain time period using a standard shaker, i.e. VARIOMAG® TELESHAKE, at a speed of 1700 revolutions per minute (rpm) and the mixing has been evaluated using a scoring of: N - not mixed; P - partly mixed; C - completely mixed.

**Table 1: Regular standardized 384 wells microplate - flat bottom**

| Dilution (µl) Solvent Solute | | Time (min) | | | | | | | | |
|---|---|---|---|---|---|---|---|---|---|---|
| | | 0.25 | 0.5 | 1 | 2 | 3 | 4 | 5 | 10 | 15 |
| 20 | 10 | N | N | P | C | C | C | C | C | C |
| 40 | 10 | N | N | N | N | N | N | P | P | P |
| 60 | 10 | N | N | N | N | N | N | N | N | N |
| 80 | 10 | N | N | N | N | N | N | N | N | N |

**Table 2: Regular standardized 384 wells microplate - rounded bottom**

| Dilution (µl) Solvent Solute | | Time (min) | | | | | | | | |
|---|---|---|---|---|---|---|---|---|---|---|
| | | 0.25 | 0.5 | 1 | 2 | 3 | 4 | 5 | 10 | 15 |
| 20 | 10 | P | C | C | C | C | C | C | C | C |
| 40 | 10 | N | P | C | C | C | C | C | C | C |
| 60 | 10 | N | N | P | P | P | P | C | C | C |
| 80 | 10 | N | N | N | N | N | N | N | P | P |

**Table 3: 384 elongated cross-section wells microplate - flat bottom**

| Dilution (µl) Solvent Solute | | Time (min) | | | | | | | | |
|---|---|---|---|---|---|---|---|---|---|---|
| | | 0.25 | 0.5 | 1 | 2 | 3 | 4 | 5 | 10 | 15 |
| 20 | 10 | C | C | C | C | C | C | C | C | C |
| 40 | 10 | C | C | C | C | C | C | C | C | C |
| 60 | 10 | C | C | C | C | C | C | C | C | C |
| 80 | 10 | P | C | C | C | C | C | C | C | C |

**Table 4: 384 elongated cross-section wells microplate - rounded and slanted bottom**

| Dilution (µl) Solvent Solute | | Time (min) | | | | | | | | |
|---|---|---|---|---|---|---|---|---|---|---|
| | | 0.25 | 0.5 | 1 | 2 | 3 | 4 | 5 | 10 | 15 |
| 20 | 10 | C | C | C | C | C | C | C | C | C |
| 40 | 10 | C | C | C | C | C | C | C | C | C |
| 60 | 10 | C | C | C | C | C | C | C | C | C |
| 80 | 10 | C | C | C | C | C | C | C | C | C |

Table 1 and table 2 show the improving mixing effect of rounded bottoms compared to flat bottoms using regular standardized microplates with wells having circular cross-sections. Table 3 if compared to table 1 and table 2 shows the tremendous reduction of time needed for efficiently mixing a liquid being arranged in a multi-well plate according to the invention with elongated cross-section wells having flat bottoms. Even compared to the regular standardized microplate having circular cross-section wells with rounded bottoms, said multi-well plate shows a significant improvement of mixing efficiency. Further, table 4 shows again a further improvement if the elongated cross-section wells are additionally arranged with rounded and slanted bottoms.

## Claims

1. Multi-well plate (1) for mixing a fluid, comprising a plurality of wells (2) each having an opening arranged at a top side of the multi-well plate (1), **characterized in that** at least one of the wells (2) has a cross-section parallel to the top side having an elongated shape.

2. Multi-well plate (1) of claim 1, wherein the cross-section has an oval shape or the shape of a rounded rectangle.

3. Multi-well plate (1) of claim 1 or 2, wherein the bottom of the at least one of the wells (2) is slanted.

4. Multi-well plate (1) of one of claims 1 to 3, wherein the bottom of the at least one of the wells (2) is rounded.

5. Multi-well plate (1) of one of claims 1 to 4, comprising 384 wells (2) arranged in 16 rows and 24 columns.

6. Multi-well plate (1) of one of claims 1 to 4, comprising 96 wells (2) arranged in 8 rows and 12 columns.

7. System for mixing a fluid, comprising the multi-well plate (1) of one of claims 1 to 5 and a mixing device arranged for shaking the multi-well plate (1).
